Europäisches Patentamt

European Patent Office

Office européen des brevets

(11) Publication number: **0 310 322**
**A2**

# EUROPEAN PATENT APPLICATION

(21) Application number: 88308915.3

(51) Int. Cl.⁴: **A61K 31/557**

(22) Date of filing: 26.09.88

(30) Priority: 30.09.87 US 102970

(43) Date of publication of application:
05.04.89 Bulletin 89/14

(84) Designated Contracting States:
AT BE CH DE ES FR GB GR IT LI LU NL SE

(71) Applicant: ALLERGAN, INC
2525 Dupont Drive
Irvine California 92715(US)

(72) Inventor: Leopold, Irving Henry
1484 Galaxy Drive
Newport Beach California 92660(US)
Inventor: Conway, Janet L.
12162 Orvillina Drive
Santa Ana California 92705(US)

(74) Representative: Giddings, Peter John, Dr. et al
Smith Kline & French Laboratories Ltd.
Corporate Patents Mundells
Welwyn Garden City Hertfordshire AL7
1EY(GB)

(54) Use of thromboxane B2 to constrict the pupil and lower intraocular pressure.

(57) This invention provides a method for affecting miosis or lowering intraocular pressure through the use of thromboxane $B_2$.

EP 0 310 322 A2

## USE OF THROMBOXANE B₂ TO CONSTRICT THE PUPIL AND LOWER INTRAOCULAR PRESSURE

### Background

This invention provides a method for constricting the pupil or lowering intraocular pressure through the use a thromboxane, particularly thromboxane $B_2$.

Miosis induced by surgical trauma can be a problem during extracupsular cataract surgery. Treatment with anticholinergic and sympatheticomimetic agents will not prevent pupil constriction during the operation. There is evidence that prostaglandins are partially responsible for this miosis (Crawford, Van Alphen, Cook, and Lands, 1978). Non-steroidal anti-inflammatory agents, such as indomethacin and flurbiprofen which inhibit the synthesis of prostaglandins have been found to prevent miosis during surgery. (Mishima and Masuda, 1977; Keulen-DeVos, Van Rij, Renardel De La Valette, and Jansen, 1983; Duffin, Camras, Gardner, and Pettit, 1982). Crawford, et. al. (1978) hypothesized that the contractile activity observed following $PGH_2$ administration was due to the enzymatic conversion of the endoperoxide to thromboxane to iris tissue. In addition, epoxymethano analogs, putative inhibitors of the conversion of $PGH_2$ to thromboxane, greatly reduced the iris response to $PGH_2$.

It has now been found that thromboxane $B_2$ will affect miosis and secondly, has been found to lower intraocular pressure.

### Scope of the Invention

This invention relates to a method for effecting miosis in a mammal, which method comprises administering to the eye thromboxane $B_2$ in an amount sufficient to effect said miosis.

In a second aspect, this invention relates to a method for lowering intraocular pressure where the method comprises administering to the eye an amount of thromboxane $B_2$ sufficient to effect a lowering of intraocular pressure.

### Specific Embodiments

Thromboxanes are naturally occurring substances synthesized by platelets, blood vessels, and in the intestines and pulmonary, renal and ocular tissues. They are known to be physiological mediators of vasoconstriction and bronchospastic conditions, as well as inducing platelet aggregation and platelet release.

It has now been discovered that the application of thromboxane $B_2$ to the eye will effect pupil constriction, miosis, and lower intraocular pressure when applied in the appropriate amounts.

The phrase "effecting miosis" means that the pupil will undergo at least a 0.5mm constriction in pupil diameter upon application of thromboxane $B_2$. The amount necessary to effect that change will vary with the method of application and can be affected by other drugs or trauma. For example, it is expected that a higher concentration of thromboxane $B_2$ will be needed if it is applied topically to an intact eye, in comparison to its application during surgery. Secondly, certain drugs dilate the pupil. If the pupil is dilated by such drugs, a greater amount of thromboxane $B_2$ may be required to effect miosis than if such drugs were not present.

The exact amount of thromboxane $B_2$ for any given situation will be some amount at least an order of magnitude greater than that normally present in ocular tissue. Such an amount can be determined by one of normal skill in the pharmacological arts. If thromboxane $B_2$ is administered during surgery, an amount in the range of approximately 50 to 500 ng applied to the open eye will effect miosis. Preferably, the thromboxane will be applied when surgery is being completed. If the application is made after the wound has been closed, or is applied to an intact eye, about 1 microgram to about 100 mg of thromboxane $B_2$ is preferred.

Effective application of thromboxane $B_2$ for effecting miosis or for the lowering of IOP can be by any number of means. However, it is most efficaciously applied directly to the eye either as a topical application in drop or ointment form or some other normal ophthalmic topical formulation. Thromboxane $B_2$ can also be administered by intracameral injection or by irrigation of the anterior chamber of the eye during surgery in the form of a isotonic fluid or gel or some similar vehicle by which the thromboxane $B_2$ is applied directly to the surgical site or sites on or in the eye exposed during such surgery. While the thromboxane $B_2$ could be administered by intramuscular injection, subcutaneously or by mouth, the desired effect of pupil constriction would be expected to be reduced. Hence, these routes of administration are not preferred for this particular application.

Pupil constriction is a useful ophthalmic technique utilized at the end of an intraocular surgery. For example, after implanting an intraocular lens, constriction of the pupil ensures the proper position of the lens will be maintained post-operatively.

Any ocular formulation such as a solution, suspension, gel, ointment, or salve and the like may be used to administer the thromboxane $B_2$. Preparation of ocular formulations are well described in the art of pharmaceutical formulations as exemplified, for example, Remington's Pharmaceutical Science, Edition 17, Mack Publishing Company, Easton, Pennsylvania. For ocular application, these compounds could also be administered as a spray, ointment, eye drops or by a device, particularly those now used for extended administration of drugs to the eye. If used during surgery, the thromboxane $B_2$ can be applied in an irrigating solution as part of an irrigating solution, or by separate application via syringe as an aqueous-based formulation. Other medicaments can be added to such formulation.

The following procedures and results are set forth to exemplify the findings supporting this invention. These examples aren't to be taken as limiting but merely illustrate the concept and its application and utility.

## Example 1

One New Zealand albino rabbit (2-4Kg) was sedated with an intramuscular injection of a 1:1 mixture of ketamine (Vetalar, Parke-Davis and xylazine (Rompum, Haver-Lockhart). The pupil diameters of both eyes were measured under standard room lighting with an optistick and recorded. The right eye was injected intracamerally with 100ng of thromboxane $B_2$ (12ul). The contralateral eye was injected with an equal volume of the vehicle solution, 0.05% BSA (Bovine Serum Albumin) in 0.01N $CH_3COOH$. Hamilton syringes with 30 gauge needles were used for the injection. Five minutes later the pupil diameters of both eyes were measured and recorded. Sequential injections of thromboxane $B_2$ (Sigma) were made into the right eye and an equal volume of vehicle into the left. Five minutes after each injection the pupil diameters were measured and recorded. A total of three injections of thromboxane $B_2$ (TXB2) were administered to the right eye; 100ng, 1ug, 10ug (12ul). After the last injection of TXB2, carbachol, 200ng (1ul) was administered to both eyes, intracamerally, and the pupil diameters were measured after five minutes.

In rabbit #8394, given three sequential doses of TXB2 in the amounts of 100ng, 1ug and 10ug, no significant changes in pupil diameter occurred following TXB2 administration. The pupils constricted an expected amount after Carbachol 200ng (1ul) injection. Refer to Table 1.

Table 1

| Rabbit # | Eye | Baseline Pupil Diameter | TXB2 Dose | Carbachol Dose | Pupil Diameter |
|----------|-----|--------------------------|-----------|----------------|----------------|
| 8394 | OD<br>OS | 6.0<br>6.5 | 100ng<br>0 | | 5.0<br>6.5 |
| 8394 | OD<br>OS | | 1ug<br>0 | | 5.0<br>6.0 |
| 8394 | OD<br>OS | | 10ug<br>0 | | 5.5<br>6.0 |
| 8394 | OD<br>OS | | | 200ng<br>200ng | 3.0<br>1.5 |

## Example 2

Seven New Zealand albino rabbits were treated, topically, with 0.03% flurbiprofen (Ocufen, Allergan) every 1/2 hour for two hours to inhibit endogenous synthesis of thromboxane. Following flurbiprofen treatment the rabbits were sedated with a 1:1 mixture of Ketamine and Rompum and the pupil diameters of both eyes were measured and recorded as before. 50ug (6ul) of TXB2 was injected intracamerally into the right eye of each rabbit and an equal volume of vehicle was administered to the left eyes. The pupil diameters were measured five minutes following the injections. This does of TXB2 was chosen because it is 1000 times the concentration of TXB2 found in clotting blood in rabbits (Ali and Mohammed, 1986). Carbachol 200ng (1ul) was injected into both eyes and the pupil diameters recorded after a five minute and a ten minute duration.

This experiment demonstrated that the pupil diameters of the TBX2 treated eyes were no different from those eyes receiving vehicle. Refer to Figure 1. Next, both eyes were injected with 200ng (1ul) of carbachol. Pupil diameter decreased significantly and to the same degree in both eyes. Neither flurbiprofen pretreatment, nor TXB2 administration affected the ability of the pupillary sphincter to contract when stimulated by carbachol, a muscarinic agonist.

### Figure 1

**Effect of Flurbiprofen, Thromboxane B2 and Carbachol on NZA Rabbit Pupil Diameter**

## Example 3

Fifteen male and female domestic cats were treated topically with 0.03% flurbiprofen (in both eyes) every 1/2 hours for two hours to inhibit endogenous thromboxane synthesis. The cats were sedated with 0.5 to 0.75ml of Rompum (20mg/ml) by intramuscular injection. Rompum was used because it does not have any effect on pupil size. The pupil diameters of both eyes were measured with an optistick and recorded.

The right eye of each cat was injected intracamerally with one concentration of thromboxane $B_2$ ranging from a high of 500ng (10ul) to a low of 10ng (10ul). The contralateral eye was injected with an equal volume of the vehicle: saline. The pupil diameters were measured five and ten minutes after the injections. The cats were allowed to regain consciousness while in the laboratory.

In this experiment, TXB2 was found to have a dose-related effect on pupil diameter in the cat. Increasing concentrations of TXB2 led to greater constriction of the pupil (miosis). Maximum constriction of the pupil occurred at a dose of 500ng. The $ED_{50}$ was 70ng. Refer to Figure 2. Figure 2 shows the effect of TXB2 on pupil diameter. The filled circles are the mean pupil diameters of the TXB2 treated eyes. The pupil diameters of the vehicle treated eyes are shown by the open circles.

## Figure 2

Effect of Thromboxane B2 on Pupil Diameter in Cats Following Flurbiprofen Pretreatment of 0.03%

## Example 4

Eight Cebus apella monkeys were treated topically with thromboxane $B_2$ in one eye and normal saline in the contralateral eye. The monkeys were awake and alert and seated in primate chairs for the duration of the experiment. The thromboxane $B_2$ concentrations used were 0.625ug, 1.25ug, 6.25ug, 12.5ug, 62.5ug, 125.0ug and 250ug per drop. A 50ul drop was administered following the baseline pupil diameter measurement. Three more drops were given at 15 minute intervals. Total dose per eye after 4 drops ranged between 2.5 micrograms and 1 mg. Pupil diameter was measured just before each administration. The last measurement was taken at 60 minutes.

In the eight Cebus monkeys with TXB2-treated eyes, the pupil diameters decreased 0.75 to 1.0 mM at 30 minutes. No change was observed in the vehicle-treated eyes. Pupil constriction of the same magnitude occurred in all TXB2 treated eyes and no dose-response relationship was observed.

The range of doses used probably represented the upper end of the dose-response curve. The 0.75 to 1.0mM pupil constriction represents a significant response in the small Cebus eye. In untreated Cebus monkey eyes, the mean pupil diameter is 4.2 ± 0.1 mm. the response to $TXB_2$ represents a 17.8 to 23.8% decrease.

## Example 5

Typical ocular formulations suitable for application of thromboxane $B_2$ are as follows:

### Ophthalmic Ointment

| | |
|---|---|
| White petroleum | 55.0 |
| Mineral oil | 42.5 |
| Nonionic lanolin derivatives | 2.0 |
| Chlorobutanol | 0.5 |
| Thromboxane $B_2$ | 5ug – 500 mg/ml |

r1029V
16570

### Balanced Salt Solution

| Ingredient | % (w/v) |
|---|---|
| Sodium Chloride | 0.64 |
| Potassium Chloride | 0.075 |
| Calcium Chloride | 0.048 |
| Magnesium Chloride | 0.03 |
| Sodium Acetate | 0.39 |
| Sodium Citrate | 0.17 |
| Thromboxane $B_2$ | 1ug – 1mg |
| Water | sufficient to make volume |

### Aqueous Topical Formulation

| | |
|---|---|
| Polyvinyl alcohol | 1.4 |
| Chlorobutanol | 0.5 |
| Sodium Chloride | 0.7 |
| Thromboxane $B_2$ | 5ug – 500 mg |
| Purified water and sodium chloride – Quantity sufficient to make volume | |

## Claims

1. A method for effecting miosis in a mammal, which method comprises administering to the eye thromboxane $B_2$ in an amount sufficient to effect said miosis.

2. The method of claim 1 where the thromboxane $B_2$ is administered during surgery.

3. The method of claim 2 where the thromboxane $B_2$ is administered in an ocular irrigating solution.

4. The method of claim 1 where the thromboxane $B_2$ is administered as a topical formulation to the eye.

5. A method for lowering intraocular pressure, which method comprises administering to the eye an amount of thromboxane $B_2$ sufficient to effect a lowering of intraocular pressure.

6. The use of thromboxane $B_2$ in the preparation of a medicament for effecting miosis in the eye of a mammal.

7. The use of thromboxane $B_2$ in the preparation of a medicament for lowering intraocular pressure.

8. A composition suitable for effecting miosis in the eye of a mammal or lowering intraocular pressure comprising thromboxane $B_2$ and a carrier or diluent therefor.

9. A composition according to claim 5 adapted for topical administration to the eye.